# EUROPEAN PATENT APPLICATION

(11) **EP 3 035 233 A1**
(43) Date of publication of application: **22.06.2016**
(21) Application number: 14198816.2
(22) Date of filing: 18.12.2014
(51) Int. Cl.: G06K 9/00, A61B 5/16

(54) **Assessment method for facial expressions**

(71) Applicant: Kobel, Paul, 8633 Wolfhausen (CH)
(72) Inventor: Kobel, Paul, 8633 Wolfhausen (CH)
(74) Representative: Schulz Junghans Patentanwälte PartGmbB

(57) **Abstract**

The invention relates to a method and computer program for evaluating faces and facial expressions comprising the steps of:
- providing an image comprising a face having a facial expression,
- detecting the face in the image,
- identifying and selecting facial components of the detected face,
- estimating at least one shape parameter for each of the selected facial components,
- assigning a value and a description to each estimated shape parameter, thereby creating a set of values and associated descriptions for selected facial components, wherein each description comprises at least one word,
- using at least one of said descriptions associated to one of the largest or one of the smallest values of the set of values and associated descriptions for evaluating the facial expression of the detected face, thereby creating an individual description.

## Description

The invention relates to a method and a computer program for characterizing faces and facial expressions.

Characterizing facial expressions is a demanding task for face detection systems or face recognition systems. State of the art systems can detect faces in many environments from different views and angles using methods with an underlying or modified so-called Viola-Jones algorithm (US7099510 B2). Methods for identifying facial expressions have already entered the consumer market and are still further developed.

However, deciphering and assessing the face and its facial expressions with respect to personal characteristics and the underlying emotions is hardly achieved by known methods.

The assessment of a face and its facial expressions is usually performed by specialists and is based on a variety of knowledge bases, such as psychology, medicine, neuroscience, phonetics, indigenous knowledge, the perception of microexpressions and other sources of personal information.

To access the above mentioned information and to sort said information is difficult, as it has to be decided how features, manifesting for example in the size and shape of facial dimensions have to be rated for a meaningful characterization.

Many approaches only use a single feature of the face or facial expression in order to assess a person's character trait and mood a person is in. That this approach is not assessing the person's mood necessarily correctly can be seen in the following example: A laughing person might not necessarily be happy.

Therefore, the problem underlying the present invention is to provide a method as well as a computer program allowing in a reliable and automated way the characterization of faces and facial expressions and potentially evaluating the person's character trait and actual mood.

This problem is solved by a method having the features of claim 1, and a computer program having the features of claim 11 described below. Preferred embodiments are stated in the sub claims.

According to claim 1, a method for evaluating face and facial expressions comprises the steps of:
- providing an image comprising a face having a facial expression,
- automatically detecting the face in the image by using for example a Viola-Jones algorithm or another face detection method,
- automatically identifying and selecting facial components of the detected face by using for example a method suited for feature detection and/or extraction, wherein the facial components comprise for example the nose, the eyes, the mouth, the lips, the cheeks etc.,
- automatically estimating at least one shape parameter for each of the selected facial components, wherein the shape parameter describes particularly the size, the shape and/or various other dimensions, such as length, width, curvature, position, or colour of the facial component,
- assigning a value and a description to each estimated shape parameter, thereby creating a set of values and associated descriptions for selected facial components, wherein each description comprises at least one word, wherein said value is particularly referenced to the average value of the associated shape parameter found in an average human population exhibiting for example a neutral facial expression,
- using at least one of said descriptions associated to one of the largest or one of the smallest values of said set (of values and associated descriptions) for evaluating the facial expression of the detected face, thereby creating an individual description of the face and/or facial expression.

The provided image particularly comprises a single image or an image from an image stack (movie) or might as well be a three-dimensional image representation comprising the face.

A person's face without any facial expression contains particularly all required information to identify and evaluate a person's current character trait and attitude in detail. The attitude of a person might be characterized with attributes like logical, emotional, materialistic etc. A facial expression furthermore reflects particularly a facial type and particularly refers to the mood, or mental state of the person additional to the underlying character trait and attitude.

In order to extract selected facial components and their corresponding shape parameters, preferably the frontal view and profile view of a face is evaluated. It might however prove advantageous to provide several images comprising the same face viewed under different angles, so that the facial components and particularly the shape parameters of the facial components can be evaluated in their entirety.

Each value of a shape parameter comprises an associated description. A description might contain words, one or more phrases, or a link referring to another facial component. This link might be used for cross-linking descriptions of selected facial components to descriptions of other facial components. Hence by combining descriptions of several facial components through cross-linking them with each other a holistic evaluation of a person's character trait can be made.

According to the invention, the largest values and smallest values are evaluated by the method, particularly as these values show the largest deviation of the average shape parameter and are therefore particularly used to find the most promising descriptions for characterizing the face or facial expression.

The magnitude of a value of a specific shape parameter is particularly estimated in relation to other values of other shape parameters, such that the values are particularly representing the relative position, the relative extent, the relative distance to other facial components, the relative size and dimension of a certain facial component with respect to other facial components.

Therefore it is possible to assign values to the shape parameters that particularly represent the relative dimensions of the shape parameters with respect to the other shape parameters of particularly the same facial component, but also in relation to shape parameters of the same or particularly a different face.

One way to estimate the dimensions, the size etc. for a specific shape parameter is to reference obtained positions, dimensions etc., that can be measured for example in units of pixels, against the size, dimensions and/or shape of the head or detected face of the person (also measured in units of pixels). In this way, it is not necessary to introduce an external ruler to the image as the obtained shape parameters are relative and not expressed in absolute units of measurement (such as pixels or millimetres).

On average the largest values are particularly given by 5 to 15, particularly, if necessary, also less or more, particularly [1,∞], largest values found for selected facial components for a detected face.

On average the smallest values are particularly the 5 to 15, particularly, if necessary, also less or more, particularly [1,∞], smallest values found for selected facial components for a detected face.

For each shape parameter the associated values are particularly predefined, wherein the magnitudes of the values depend on the specific shape parameter. The following table illustrates as for an example the association between facial components, shape parameter and possible associated values:

| **facial component** | **shape parameter** | **Value** | |
|---|---|---|---|
| nose | length | 0 | (short) |
| | | 0,5 | (middle) |
| | | 1 | (long) |
| | width | 0 | (slim) |
| | | 0,5 | (middle) |
| | | 1 | (wide) |
| | tip | 0 | (pointed) |
| | | 0,5 | (round) |
| | | 0,8 | (fitted) |
| | | 1 | (split) |
| lips | width | 0,1 | (very narrow) |
| | | 0,4 | (narrow) |
| | | 0,75 | (wide) |
| | | 1 | (very wide) |
| cheeks | distinction | 0 | (not distinct) |
| | | 0,5 | (average) |
| | | 1 | (very distinct) |

Some shape parameters might require personal information, if the analysis results are not clear e.g. after a plastic surgery or mutation. These facial components might be excluded from the face evaluation or awarded with a high or low value.

The associated descriptions of the values are stemming from a first-knowledge database with is acquired and collected from available books and databases from several disciplines such as medicine, psychology, neuroscience, indigenous knowledge. For example the description associated to a high value of the shape parameter (width) of the lips can comprise the words "emotional, sensible", whereas the description associated to a high value of the shape parameter (distinction) of the cheeks can comprise the words "sensitive, sensible".

The description associated to the value of the shape parameters of all or selected facial components, particularly the descriptions associated to the largest and smallest values, are used to evaluate face and the facial expression and to generate the individual description of face and the facial expression.

In one embodiment of the method the individual description comprises at least a or several words, a phrase or a sentence.

One way to generate the individual description for example is to combine, and display the words of said descriptions of the largest and smallest values. The display can for example emphasize descriptions that are considered to be most informative regarding the characterization of a person's face and facial expression. As each description comprises at least one word one can for example display the individual description in a so-called word-cloud, often found on websites e.g. to display topics most common looked for.

In another embodiment of the invention the individual description is organized as a table. A table is any kind of ordered representation of the words used for evaluating the facial expression and the corresponding facial component or shape parameter said words are associated to.

As it is possible to generate for example a phrase, a sentence or a plurality of sentences using a semantic method or tool, the individual description can be generated by such a method that generates such phrase or sentence(s) particularly from the words of the descriptions used for the evaluation of the facial expression.

In another embodiment of the invention the set of values and the associated descriptions is sorted according to the magnitude of the values, wherein particularly the values exhibiting the largest and smallest magnitude are sorted to the top of the set of values and descriptions, thereby creating a sorted set of values and associated descriptions.

According to one aspect of the invention, the individual description of the facial expression of the detected face is generated using the at least one word of the at least one description. The advantages of this embodiment have already been disclosed above.

In another embodiment the words comprised by the individual description are sorted according to their relative occurrence or recurrence, wherein particularly words with the highest occurrence or recurrence are sorted to the top of a table representing the individual description.

In this way an additional characterization is facilitated, as particularly words referring to similar characteristics of descriptions of the face that have a high occurrence can be emphasized based on their relative occurrence. For example if the descriptions of the shape parameters for the selected facial components used for evaluating the facial expression comprise the word "emotional" three times and "logical" only once, than the word "emotional" is emphasized with regard to the word "logical". This can for example be achieved by representing the word "emotional" in bold letters or with a larger font size as for example the word "logical".

In another embodiment of the invention there are at least three different magnitudes of values provided for each shape parameter, wherein each value particularly ranges between 0 and 1. This embodiment is particularly advantageous as fewer magnitudes of values are likely to lead to a too coarse of a classification for the facial components for generating a meaningful analysis of the facial expression.

In another embodiment of the invention at least one, particularly at least two, particularly at least 10, particularly at least 15 facial component(s) are identified, when a face is identified in the image. The method according to the invention performs more robust the more facial components can be obtained and analysed. It is thought that 15 facial components are sufficient to obtain a satisfying characterization of the facial expression, i.e. ∼80% of assessments lead to a correct result.

In another embodiment at least three descriptions are used for generating the individual description.

Furthermore, the problem according the invention is solved also by a computer program according to claim 11.

According to claim 11, a computer program, particularly stored on a non-transitory medium, which, when executed by a computer, performs at least the steps of:
- automatically detecting a face comprised in a provided image comprising the face,
- automatically identifying and selecting facial components of the detected face,
- automatically estimating at least one shape parameter for each of the selected facial components,
- assigning a value and a description to each estimated shape parameter, thereby creating a set of values and associated descriptions for selected facial components, wherein each description comprises at least one word, and
- using at least one of said descriptions associated to one of the largest or one of the smallest values of said set (of values and associated descriptions) for evaluating the facial expression of the detected face.

Preferably, the computer program is adapted to perform any of the method steps stated in claims 2 to 10 or described herein.

As mentioned above, by using state of the art classifiers and feature detection and extraction computer programs, it is possible to execute the method according to the invention on a computer, or a computer-like device, particularly when the computer program according to the invention is stored and executed on the computer or the computer-like device.

According to a further aspect of the present invention the following method or computer program is disclosed:
Method or computer program stored on a non-transitory medium which, when executed by a processor, performs the method for evaluating facial expressions, comprising at least the steps:
   - providing an image comprising a face having a facial expression,
   - automatically detecting a face comprised in the provided image,
   - automatically identifying and selecting facial components of the detected face,
   - estimating at least one shape parameter for each of the selected facial components,
   - assigning a value to each shape parameter, thereby generating a set of values for selected identified facial components,
   - compare the generated set of values with predefined sets of values for said selected facial components, wherein at least one of the predefined sets of values is assigned to each facial expression, and wherein each facial expression is characterized by a description, wherein the description contains at least one word,
   - evaluating the facial expression from the detected face by assigning the generated set of values to a facial expression whose corresponding at least one predefined set of values is closest to the generated set of values, and by assigning the description of the facial expression whose corresponding at least one predefined set of values is closest to the generated set of values to the detected facial expression, by means of a classifier machine.

Such classifier machines are known from the state of the art. Known classifiers are for example a multiclass-support vector machine or a cascade classifier.

The closest distance if such sets is defined by the specific classifier used. Using for example a support vector machine classifier, the distance is the measured from a specific hyperplane defined by the predefined sets of values. Using a support vector machine also defines a closest distance by the metric used of the underlying support vector machine.

Further features and advantages of the invention shall be described by means of a detailed description of embodiments with reference to the figures, wherein
- Fig. 1: shows a flow-chart describing the method according to the invention; and
- Fig. 2: shows a frontal view of a face.

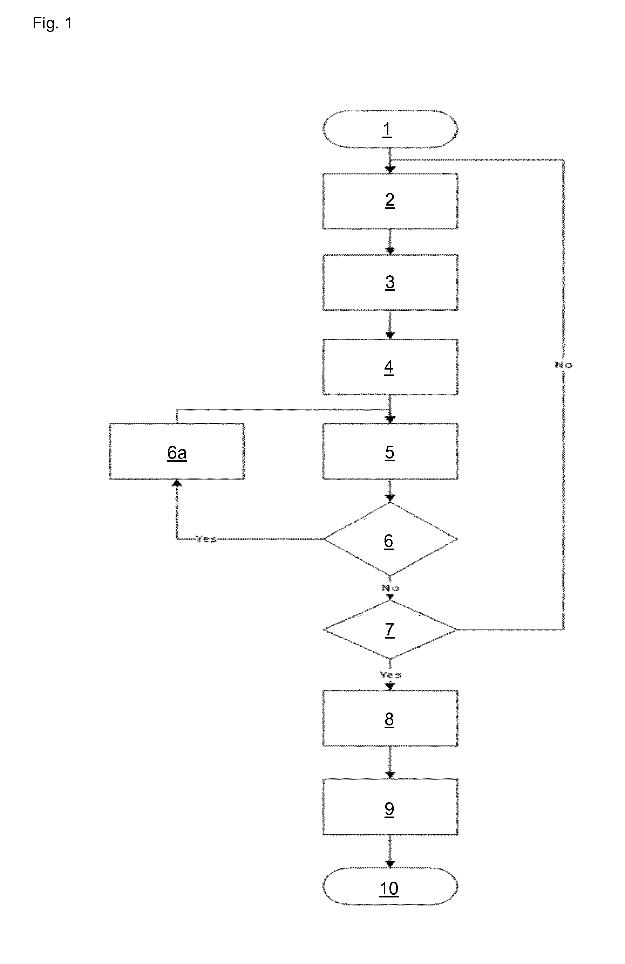

Fig. 1 shows a flow-diagram of the method or computer program according to the invention. The method is started or the computer program is executed 1. An image, an image stack or similar means comprising a face having a facial expression are provided and the face is detected 2. The proposed method detects a face by use of a component-based classifier with a single support vector machine (SVM). Proposed by e.g. Heisele, B., Poggio, T. and Pontil, M., (2000), "Face detection in still ridge 'Images', 1 A. memo 1687, Center for Biological and Computational Learning, MIT, Cambridge, MA. The basic calculation of the component-based single SVM classifier aims to detect faces of different sizes and arbitrary positions in a gray-scale input image. To do this, a window of fixed size is shifted over each of the scaled images. The pattern within each window is analysed by a two-level component-based classifier, which has a binary output indicating that a face is located in the window or not.

In a third step 3, facial components and facial features are extracted from the image. In short, this stage of classification independently identifies each facial component. The SVM classifier is also used to detect each facial component, such as eye, nose and mouth. The facial component consists of three sub-steps. First, a generation step generates facial component information data. Next, the facial components are evaluated by a judging step as being suitable or not. Then, in a facial component identification step the facial components information that is stored for example in a memory means are identified. The identification of the facial components underlies a feature recognition/detection module. The feature recognition/detection module is for example a trained classifier based on training images for each facial component provided by a camera or an image data database.

In a fourth step 4, positions and distances between the facial components respectively specific reference points of the facial components (e.g. distance between the eyes, distance between the ears, distance between ear and nose tip...) are evaluated. The shape parameters such as for example, the dimensions of the facial component are measured absolutely, for example in units of pixels. Then the absolute distances and all measured absolute dimensions/specifications are weighted and transferred into a relative value by setting them in relation to each other. This value allows comparing facial component from different persons. For example a big nose is then identified as big nose, when its proportion (defined by its specific shape parameters) relating to other facial components is larger than the mean/average proportion of the nose in the data base. The measuring process refers to a checklist/set of facial component or and/or reference points such as:
1. Head, face, hair, forehead, eyebrow, nose, lip, heart of the lip, cheeks, ears, neck,
2. Wrinkles: Forehead wrinkles, nasolabial folds, chink wrinkles, nose wrinkles, ear wrinkles, eye wrinkles, nose wrinkles (all horizontal and vertical),...
3. Hands, teeth, eyes, eyes length with characteristics thick, thin, round, edgy, full, empty,...

This checklist of facial components and reference points can further be complimented with body components such as arms, legs, feet, torso,...

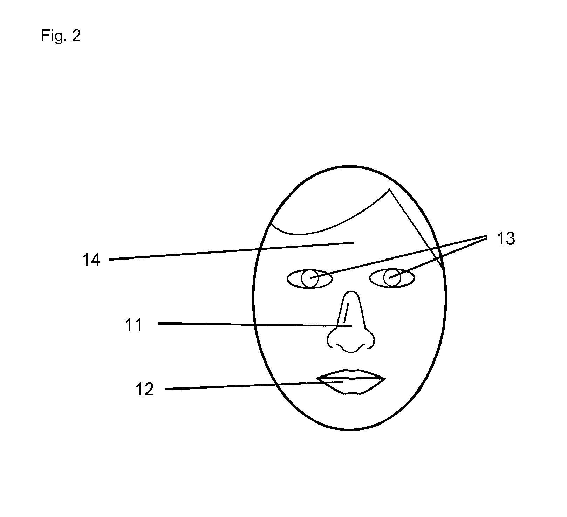

The shape parameters such as height, wideness, length, bulge, depression, colouring, blood circulation, movement, body attitude, gesture and mimicry, posture, sensual emotion, sound (if possible), temperature, frequencies of an individual, are part of the analysis and evaluation. Each above mentioned facial component/reference point is characterized by the shape parameters and the assigned values (see Fig. 2), step 5.

If this is not possible or if there are arbitrary or contradicting results, personal information might be obtained 6, 6a.

A specific description is associated 7 to each value that derives from the first-knowledge base. The descriptions refer to personal attitudes and characteristics and are predefined and particularly independent of the face to analyse.

The estimated, particularly relative, value is then particularly matched (e.g. by rounding up or down) with a predefined value with an associated description. This description for said value is memorized in a data base for every selected facial component.

By combining the different descriptions associated to the values of the shape parameters of the facial components, from the image, a personal, individual description of the face on the image is derived 9.

The results of the facial component evaluation are put in an order (step 8) according to their respective importance in the specific case. Extreme values of the shape parameters are ranked higher in the order. The order allows to cross-link the descriptions. The descriptions can mainly be distinguished into different classes (e.g. emotional type, logical type, materialistic type...). By combining single descriptions through the given order and considering the occurrence of words comprised in the descriptions, it is possible to generate far better descriptions of the personal character than by only putting them together.

By saving the results in a data base it is further possible to achieve information learning from past results. Therefore the following algorithm is used for the SVM.

In a last step 9 the facial expression is evaluated for example by generating an individual description form all words of descriptions used in the evaluation of the facial expression. The method is then completed 10.

For the whole method there needs to be clarity around each facial component. Furthermore the facial component might need to be broken down into more detailed specification if necessary to capture the specific facial component most accurately and correctly. Therefore each reference point in itself has a further own complexity. Besides clarity and detail for the facial components, there is a measure of importance for each facial component and additional special features that add to the ability to learn as a system adding to the knowledge pool of experience offering an additional combination of cross-linked formations involved.

Fig. 2 shows a frontal view of an image comprising a face having a facial expression. Exemplarily the nose 11, the lips 12, the cheeks 13 and the eyebrows 14 are marked as facial components.

## Claims

**1.** Method for evaluating faces or facial expressions comprising the steps of:
- providing (1, 2) an image comprising a face having a facial expression,
- detecting (2) the face in the image,
- identifying and selecting (3) facial components of the detected face,
- estimating (4) at least one shape parameter for each of the selected facial components,
- assigning a value (5, 7) and a description to each estimated shape parameter, thereby creating a set of values and associated descriptions for selected facial components, wherein each description comprises at least one word,
- using (9) at least one of said descriptions associated to one of the largest or one of the smallest values of said set for evaluating the facial expression of the detected face, thereby creating an individual description of the facial expression.

**2.** Method according to claim 1, **characterized in that** the individual description comprises a word, a phrase or a sentence.

**3.** Method according to claim 1 or 2, **characterized in that** the individual description is organized as a table, a NoSQL database or another particularly machine-searchable database.

**4.** Method according to one of the preceding claims, **characterized in that** said set is sorted (8) according to the magnitude of the values, wherein the values exhibiting the largest and smallest magnitude are sorted to the top of said set, thereby creating a sorted set of values and associated descriptions.

**5.** Method according to one of the preceding claims, **characterized in that** the individual description of the facial expression of the detected face is generated using the at least one word of the at least one description.

**6.** Method according to one of the preceding claims, **characterized in that** the words comprised by the individual description are sorted according to their relative occurrence.

**8.** Method according to one of the preceding claims, **characterized in that** there are at least three different magnitudes of values provided for each shape parameter, wherein each value particularly ranges between 0 and 1.

**9.** Method according to one of the preceding claims, **characterized in that** at least one facial component is identified, when a face is identified in the image.

**10.** Method according to one of the preceding claims, **characterized in that** at least three descriptions are used for generating the individual description.

**11.** Computer program comprising program code for executing the following steps when the computer program is executed (1) on a computer:
- detecting (2) a face comprised in a provided image comprising the face,
- identifying and selecting (3) facial components of the detected face,
- estimating (4) at least one shape parameter for each of the selected facial components,
- assigning (5, 7) a value and a description to each estimated shape parameter, thereby creating a set of values and associated descriptions for selected facial components, wherein each description comprises at least one word,
- using (9) at least one of said descriptions associated to one of the largest or one of the smallest values of said set for evaluating the facial expression of the detected face, thereby creating an individual description of the facial expression.
